(19) Europäisches Patentamt
European Patent Office
Office européen des brevets

(11) Publication number : **0 572 122 A2**

# (12) EUROPEAN PATENT APPLICATION

(21) Application number : **93303420.9**

(22) Date of filing : **30.04.93**

(51) Int. Cl.[5] : **C07K 7/00,** G01N 33/68, G01N 33/574, A61K 37/02

The applicant has subsequently filed a sequence listing and declared, that it includes no new matter.

(30) Priority : **30.04.92 IL 101747**

(43) Date of publication of application : **01.12.93 Bulletin 93/48**

(84) Designated Contracting States : **AT BE CH DE DK ES FR GB GR IE IT LI LU MC NL PT SE**

(71) Applicant : **YISSUM RESEARCH AND DEVELOPMENT COMPANY OF THE HEBREW UNIVERSITY OF JERUSALEM**
**46 Jabotinsky Street**
**Jerusalem 91 999 (IL)**

(72) Inventor : **Bab, Itai**
**26 Hateena Street**
**Carmei Yosef, M.P. Ayalon (IL)**
Inventor : **Shteyer, Arie**
**37 Haarazim Street**
**Mevasseret Zion (IL)**
Inventor : **Muhlrad, Andras**
**29/A Nave Shaanan Street**
**Jerusalem (IL)**
Inventor : **Chorev, Michael**
**135/4 Feinstein Street**
**Jerusalem (IL)**
Inventor : **Gazit, Dan**
**47 Peretz Bernstein Street**
**Jerusalem (IL)**
Inventor : **Greenberg, Zvi**
**19 Joshua Ben-Nun Street**
**Givat Zeev. (IL)**

(74) Representative : **Goldin, Douglas Michael et al**
**J.A. KEMP & CO. 14, South Square Gray's Inn**
**London WC1R 5LX (GB)**

## (54) C-terminal modified osteogenic growth polypeptides.

(57) The invention relates to a polypeptide which binds to naturally occurring osteogenic growth-polypeptide binding protein/s (OGPBP) and does not bind to an antibody directed against said naturally occurring osteogenic growth polypeptide (OGP), said OGPBPs being proteins which bind to the N-terminal region of OGP and to analogues thereof.

Preferred polypeptides according to the invention are those in which the C-terminal region is modified to prevent binding of the polypeptide to an antibody directed against OGP.

Particular polypeptides according to the invention are [$Cys^{15}$ (NEM)]OGP-$NH_2$ and [$Tyr^{10}$ (3-I)]OGP.

The invention also relates to a method of releasing an immunoreactive OGP from its complex with OGPBPs wherein the said complex is reacted with a polypeptide according to the invention, whereby the immunoreactive OGP is competed out of said complex by the polypeptide according to the invention.

The invention further relates to a method of quantitatively determining the total immunoreactive OGP content in a sample of a biological fluid or tissue extract and to a method for the determination of the binding capacity of OGPBPs.

The methods of the invention are useful for diagnosing pathological conditions associated with abnormal binding capacity of OGPBPs by measuring the said binding capacity. Particular pathological conditions are such which are associated with abnormal bone formation and various metabolic bone diseases e.g., osteoporosis, hyperparathyroidism, osteomalacia, rickets or renal osteodystrophy.

The invention also relates to pharmaceutical compositions containing as active ingredient polypeptides according to the invention.

EP 0 572 122 A2

## FIELD OF THE INVENTION

The invention relates to polypeptides capable of competing out osteogenic growth polypeptides from their complexes with osteogenic growth polypeptide-binding proteins as well as their therapeutic and diagnostic applications.

## BACKGROUND OF THE INVENTION

It has been established that regenerating marrow induces an osteogenic response in distant skeletal sites and that this activity is mediated by factors released into the circulation by the healing tissue [Bab I., et al. (1985) Calcif. Tissue Int. 37:551; Foldes, J., et al. (1989) J. Bone Min. Res. 4:643; Einhorn, T.A., et al. (1990) J. Bone Joint Surg. Am. 72:1374; Gazit, D., et al. (1990) Endocrinology 126: 2607; Mueller, M., et al.(1991) J. Bone Min. Res. 6:401]. One of these factors, a 14-amino acid osteogenic growth polypeptide, identical with the C-terminus of histone H4 (HH4), has been recently identified [European Patent Applications Nos. 89201608.0 and 90301862.0].

Synthetic osteogenic growth polypeptide, identical in structure with the native molecule, has been shown to be a potent stimulator of proliferation of osteoblastic and fibroblastic cells in vitro. This synthetic polypeptide also stimulates osteoblastic cell alkaline phosphatase activity. When injected in vivo to rats, at very small doses, the synthetic osteogenic growth polypeptide increases bone formation and trabecular bone mass.

As in the case of other polypeptide growth regulators, such as growth hormone and insulin-like growth factor [Hintz, R.L. (1990) Horm. Res. 33:105], osteogenic growth polypeptide - binding protein/s may protect the osteogenic growth polypeptide against proteolytic degradation, thus regulating its active level. However, the presence of a binding protein with high affinity to the peptide is of great concern when immunochemical assays are performed [Daughaday, W.H., et al. (1986) J. Lab. Clin. Med. 109:355]. Binding proteins can block the epitope against which the antibodies are directed, so that only a portion of the peptide factor is measured [Daughaday, W.H., et al. (1980) Clin . Endocrinol. Metab. 51:781]. In addition, the binding protein(s) compete with the antibodies for the ligand, resulting in falsely measured concentrations [Blum, W.F., et al. (1988) acta Endocrinol. (Copenh) 118:374].

Quantitative determination of the total immunoreactive osteogenic growth polypeptide in body fluids, as well as the steady state osteogenic growth polypeptide and the binding capacity of the osteogenic growth polypeptide-binding proteins, may be of diagnostic and therapeutic importance in several pathological conditions, particularly conditions involving abnormal bone formation. In addition, such quantitative determination is of analytical importance in studies on the biology of the osteogenic growth polypeptide.

However, since a major part of the immunoreactive osteogenic growth polypeptide in body fluids is bound in complex with the osteogenic growth polypeptide - binding proteins, the total immunoreactive osteogenic growth polypeptide can only be quantified if the complex is disintegrated, or if the osteogenic growth polypeptide is competed out of its complex with the osteogenic growth polypeptide - binding protein/s. Chemically or otherwise induced disintegration of the complex may affect the immunological, and possibly other, properties of the osteogenic growth polypeptide, which may prevent quantitative determination by known immunochemical techniques. Therefore, competitive release of the osteogenic growth polypeptide from its complex with the osteogenic growth polypeptide - binding proteins would appear to be preferred.

The present invention indeed relates to compounds which are capable of competing out the osteogenic growth polypeptide from its complex with osteogenic growth polypeptide - binding proteins without interfering with the quantitative determination of the total immunoreactive osteogenic growth polypeptide by immunochemical techniques.

The following abbreviations will be used hereafter:

| | |
|---|---|
| OGP(s) - | osteogenic growth polypeptide(s); |
| OGPBP(s) - | osteogenic growth polypeptide binding protein(s); |
| irOGP - | immunoreactive OGP; |
| sOGP - | synthetic OGP; |
| msOGP - | modified synthetic OGP; |
| rOGP - | retro-OGP, a synthetic peptide presenting the reverse sequence of OGP. |

## SUMMARY OF THE INVENTION

The invention relates to polypeptides which bind to naturally occurring OGPBPs and do not bind to antibodies directed against OGP or sOGP, the OGPBPs being proteins which bind to the N-terminal region of OGP and to its analogues.

The invention also relates to methods of releasing OGP from its complex with an OGPBP by contacting the complex with a polypeptide of the invention, under suitable conditions, whereby the irOGP is competed out of said complex, without interference with quantitative determination of the total irOGP by immunochemical techniques.

Additionally, the invention relates to a method of quantitatively determining total irOGP in biological fluids and tissues, comprising the steps of reacting a sample of a biological fluid or tissue extract containing OGP and OGPBP/s with a polypeptide of the invention, incubating the reaction mixture with an antibody directed against OGP, and calculating the amount of total irOGP which was present in the sample from the amount of free or bound antibody as measured by respectively suitable immunoassay techniques.

The polypeptides of the invention and the methods employing the same may be used for diagnostic and analytical purposes.

In addition, the invention relates to a method for the determination of the binding capacity of OGPBP/s quantitatively determining the total amount of irOGP in an aliquot of a sample of a biological fluid or a tissue extract by the method of the invention, incubating other aliquots of said sample each with a gradually increasing concentration of sOGP and quantitatively determining the amount of irOGP therein by immunoassay techniques and calculating the binding capacity of the OGPBP/s in said sample by subtracting, for each of the said aliquots, the amount of irOGP from the sums of the total irOGP determined and the amount of sOGP added, wherein the maximal value obtained among said aliquots by said calculation represents the binding capacity of the OGPBP which was present in said sample.

Furthermore, the invention relates to pharmaceutical compositions containing as active ingredient a polypeptide of the invention, in a pharmaceutically acceptable carrier. The polypeptides of the invention and the pharmaceutical compositions containing them may be used for treatment of various pathological conditions, particularly conditions involving abnormal bone formation.

## BRIEF DESCRIPTION OF THE FIGURES

**Figure 1** shows purification of [$Cys^{15}$(NEM)]OGP-$NH_2$ by HPLC.

(a) separation on Vydac C18 preparative reverse-phase column. Aliquots of selected fractions of the major peak (retention time 107-124 min.) were subjected to analytical reverse-phase HPLC.

(b) Elution position of [$Cys^{15}$]OGP-$NH_2$ (unreacted with NEM) on analytical RP-18 reverse-phase column.

(c) Elution position of [$Cys^{15}$(NEM)]OGP-$NH_2$ on analytical RP-18 reverse-phase column.

(d,e) Reverse-phase HPLC on analytical RP-18 column of fraction samples selected from the major peak of the preparatory separation shown in (a). Fractions 9-12 contained the [$Cys^{15}$ (NEM)]OGP-$NH_2$ purified to homogeneity as revealed by comparison to the elution profile in (b). Other fractions, including those shown in (e), were discarded.

**Figure 2** shows removal of unreacted iodide from sOGP radioiodination reaction mixture by SEP-PAK C18. Fractions 12-14 were pooled and further purified by reverse-phase HPLC (see below description of Fig. 3).

**Figure 3** shows purification of radioiodinated sOGP by HPLC. Dashed line represents light absorbance recorded during test separation of aliquot sample containing 1 mg/ml nonradioactive [$Tyr^{10}$(3-I)]OGP. Continuous line represents radioactivity of the main purification run. Fractions 37 and 38 were pooled and used as the monoradioiodinated OGP.

**Figure 4** shows an autoradiograph of cathodic native PAGE demonstrating binding of OGP to OGPBP.

**Lane a,** [$Tyr^{10}$(3-$^{125}$I)]sOGP alone;

**Lanes b-j,** [$Tyr^{10}$ (3-$^{125}$I)]sOGP preincubated with 1:50 dilution of fresh rat serum;

**b,g:** in absence of sOGP analogues;

**c:** in the presence of 0.625 nmol/ml sOGP;

**d:** in the presence of 0.625 nmol/ml rOGP

**e,h:** in the presence of 6.25 nmol/ml sOGP;

**f:** in the presence of 6.25 nmol/ml rOGP;

**i:** in the presence of 6.25 nmol/ml [Ac-$Cys^0$(NEM)]OGP; or

**j:** in the presence of 6.25 nmol/ml [$Cys^{15}$(NEM)]-OGP-$NH_2$.

**Figure 5** demonstrates the determination of the binding capacity of the OGPBP to human serum. The bars indicate irOGP measured for each concentration of sOGP added. Numbers above bars represent results of the calculation (total irOGP + added sOGP - irOGP measured for each concentration of sOGP added). The binding capacity is the maximal value resulting from this calculation for a given specimen.

**Figure 6** shows standard curve of competitive ELISA for determination of irOGP.

**Figure 7** shows competitive release of irOGP from OGP-OGPBP complex present in biological fluids, using [$Cys^{15}$(NEM)]OGP-$NH_2$:

**A)** human serum;
**B)** rat serum;
**C)** mouse serum;
**D)** F-12 medium-from ROS 17/2.8 culture 24 h after washing of cells;
**E)** αMEM medium from MC 3T3 E1 culture 24 h after washing of cells;
**F)** αMEM medium from NIH 3T3 culture 24 h after washing of cells.

**Figure 8** shows post-ablation steady state (A) and total (B) serum irOGP in individual rats. Respective symbols in both panels indicate measurements from same serum sample. P, pre-ablation levels.

**Figure 9** shows post-irradiation steady state and total serum irOGP in mice. C, control levels measured in sera from normal mice.

**Figure 10** shows effect sOGP administered intravenously on steady state and total irOGP in sera obtained from mice subjected to total body irradiation and marrow transplantation:

**group 1:** serum from control mice that received peptide-free PBS;
**group 2:** serum from mice that received sOGP three days prior to irradiation and transplantation;
**group 3:** serum from mice that received sOGP for nine days starting on day 3 prior to irradiation and transplantation.

**Figure 11** shows regression analysis between total serum irOGP and spleen cellularity in irradiated mice that received sOGP and marrow transplants.

## DETAILED DESCRIPTION OF THE INVENTION

OGP is a 14-residue polypeptide identified from regenerating bone marrow which has been shown to stimulate the proliferation and alkaline phosphatase activity of osteoblastic and fibroblastic cells in vitro and to increase bone formation and trabecular bone mass in rats when injected in vivo. The amino acid sequence of OGP is as follows:

Ala-Leu-Lys-Arg-Gln-Gly-Arg-Thr-Leu-Tyr-Gly-Phe-Gly-Gly

Synthetic OGP, with an identical amino acid sequence, has been prepared by standard solid phase methodology [Barany, G. and Merrifield, R.B. (1979) In Gross, E. and Mierhofer, J. (Eds.): The Peptides, Vol. 1, Academic Press, New York, pp.1-27]. OGP is a subject of European Patent Applications Nos.89201608.0 and 90301862.0.

It has now been found that in different biological fluids OGP forms a complex with OGPBP(s) with a role for the OGP N-terminal region in the complex formation and that C-terminal modified synthetic OGP analogues fully retain the binding properties of OGP to the OGPBP, but at the same time fail to react with anti-OGP antibody preparations, and furthermore, that the OGPBP complex also fails to react with the anti-OGP antibody preparations.

Thus, sOGP analogues modified at their C-terminal region, at sufficient concentrations, can be used to competitively release the total irOGP from OGP-OGPBP complexes, for analytical and diagnostic purposes, without interfering with the quantitative detemination of the total irOGP by immunochemical techniques. Furthermore, these novel synthetic modified OGP analogues can also have therapeutic applications, elevating the level of available OGP by competing it out from its complex with the OGPBPs.

Thus, the present invention relates to a polypeptide which binds to a naturally occurring OGPBP and does not bind to an antibody directed against said naturally occurring OGP or sOGP, said OGPBPS being proteins which bind to the N-terminal region of OGP and to analogues thereof.

More specifically, the polypeptides of the invention bind to the OGPBPs through their N-terminal region.

Preferably, the polypeptides of the invention are modified OGPs, the modification being in the C-terminal region, to prevent binding of the polypeptide to an antibody directed against OGP or sOGP. More preferably, the said antibody binds to only the C-terminal region of OGP.

Preferred polypeptides according to the invention are the following modified OGPs:

Ala-Leu-Lys-Arg-Gln-Gly-Arg-Thr-Leu-
Tyr-Gly-Phe-Gly-Gly-Cys(NEM)-$NH_2$

(wherein NEM signifies N-ethyl maleimide) (hereafter referred to as [$Cys^{15}$(NEM)]OGP-$NH_2$); and

Ala-Leu-Lys-Arg-Gln-Gly-Arg-Thr-Leu-
Tyr(3-I)-Gly-Phe-Gly-Gly

(hereafter referred to as [$Tyr^{10}$(3-I)]OGP).

Particularly preferred polypeptides according to the invention bind to OGPBPs and do not bind to an antibody directed against OGP, said antibody binding to [Ac-$Cys^0$-(NEM)]OGP-$NH_2$ (wherein NEM signifies N-ethyl maleimide) but does not bind to [$Cys^{15}$(NEM)]OGP-$NH_2$ and [$Tyr^{10}$(3-I)]OGP.

The preparation of the polypeptides of the invention is described in Example 1 (under Methods).

The polypeptides of the present invention are capable of competing out the OGP from its complex with OGPBPs without interfering with the quantitative determination of the total irOGP by immunochemical techniques.

Indeed, as will become apparent from the following Examples, the present findings establish that (i) in different biological fluids OGP forms a complex with OGPBP(s) with a role for the OGP N-terminal region in the complex formation; (ii) C-terminal modified synthetic OGP analogues fully retain the binding properties of OGP to the OGPBP, but at the same time fail to react with a polyclonal rabbit anti-OGP antibody preparation; and (iii) the OGPBP complex also fails to react with the anti-OGP antibody preparation.

The novel msOGP analogues, particularly those modified at their C-terminal region, at sufficient concentrations, can thus be used to competitively release the total irOGP from OGP-OGPBP complex(es), for analytical and diagnostic purposes, without interfering with the quantitative determination of the total irOGP by immunochemical techniques.

The invention also relates to a method of releasing irOGP or naturally occurring analogues thereof from a complex with an OGPBP, by reacting the complex with a polypeptide of the invention, whereby the irOGP or its analogue is competed out of the complex by the polypeptide of the invention, while the immunoreactivity of the OGP or its analogue is preserved.

The said preferred polypeptides of the invention, [$Cys^{15}$(NEM)]OGP-$NH_2$ and [$Tyr^{10}$(3-I)]OGP are particularly suitable for this method.

In addition, the invention relates to a method of quantitatively determining the total irOGP content in a biological fluid or tissue, comprising the steps of (a) reacting a sample of said biological fluid or tissue extract with a polypeptide of the invention under suitable reaction conditions; (b) subsequently incubating the reaction mixture obtained in step (a) with an antibody directed against OGP under suitable incubation conditions; and (c) calculating the total amount of irOGP which was present in said sample from the amount of free or bound antibody as measured by respectively suitable immunoassay techniques.

The preferred polypeptides of the invention described above are particularly suitable for the reaction in step (a). Preferred antibodies for step (b) are those prepared by using OGP or sOGP conjugated to a carrier via its N-terminal region. Various known immunoassay techniques can be employed for step (c). These include, for example, ELISA, RIA and fluorescent immunoassay. Specific immunoassay techniques, as known in the art, may be chosen for direct determination, i.e. determination of the bound antibody, or indirect determination, i.e. determination of the free antibody.

The quantitative determination of irOGP is described in detail in Example 2.

In addition, the method of quantitatively determining total irOGP content in biological fluids or tissues according to the invention enables to determine the binding capacity of the OGPBPs present in the biological fluid or tissue. Total irOGP in a an aliquot of a sample of the biological fluid or tissue extract is determined according to the method of the invention. Increasing concentrations of sOGP are incubated with further aliquots of said sample and the irOGP is measured by immunoassay techniques. The binding capacity is calculated according to the equation:

Binding capacity = Max[Total irOGP + added sOGP - measured irOGP]

The binding capacity is the maximal value resulting from this calculation for a given specimen. The determination of the binding capacity is described in detail in Example 2 and Fig. 5.

The methods of the invention thus enable to effectively and reliably determine differentially the steady state irOGP, the total irOGP and the binding capacity of OGPBPs in biological fluids, such as serum and urine, and tissues, such as bone cells. These measurements provide highly sensitive means for the diagnosis of various pathological conditions, particularly conditions involving abnormal bone formation, and more particularly metabolic bone disease such as osteoporosis, hyperpara-thyroidism, osteomalacia, rickets and renal osteodystrophy. The quantitative measurements according to the invention can also be used for the early detection of osteogenic metastases, characteristic of prostate carcinoma.

The differential determinations of the steady state irOGP, total irOGP and the binding capacity of OGPBP can also provide indications for the application of different treatment modalities, as well as monitoring the effect of the elected treatment modalities. In addition, the determination of said parameters can be useful in evaluating the viability of bone marrow and the prognosis of bone marrow transplants.

The determination of the steady state and total irOGP can also be applied to laboratory experiments where the biology of OGP is studied, in particular the screening of chromatography fractions and determination of OGP content in tissue culture media. The said measurements can also be applied to the study of the OGP binding to its receptor, in particular the role of the OGPBP in the interaction between OGP and the receptor at the experimental level, and also at therapeutic levels.

Furthermore, the novel synthetic modified OGP analogues can also have therapeutic applications, elevating the level of available OGP by competing it out from its complex with OGPBP.

The invention thus also relates to pharmaceutical compositions containing as active ingredient a polypeptide of the invention in a pharmaceutically acceptable carrier.

In the various body tissues or fluids at least a part, and possibly a major part, of biologically active OGP is complexed with OGPBPs, in which form it is inactive. The polypeptides of the invention and the pharmaceutical compositions containing the same can be used therapeutically for the competitive release of OGP from the OGP-OGPBP complex, thus increasing the levels of effective available OGP in the body. This application of the polypeptides of the invention and the pharmaceutical compositions containing them can be particularly useful for the fine tuning of OGP levels in pathological conditions characterized by increased binding capacity of the OGPBPS. The polypeptides of the invention can also be therapeutically applied in pathological conditions involving a decrease in OGP or OGPBP levels.

The magnitude of a therapeutic dose of a polypeptide of the invention will, of course vary with the group of patients (age, sex, etc.), the nature of the condition to be treated and with the particular polypeptide employed and its route of administration. In any case the therapeutic dose will be determined by the attending physician, who may be assisted by results of the quantitative determinations of the steady state and total irOGP and binding capacity of the OGPBP, as described above.

Any suitable route of administration may be employed for providing a mammal, especially a human, with an effective dosage of a polypeptide of this invention. Intravenous administration may be preferred.

The pharmaceutical compositions of the invention can be prepared in dosage units forms. The dosage forms may also include sustained release devices. The compositions may be prepared by any of the methods well-known in the art of pharmacy.

The pharmaceutical compositions of the invention comprise as active ingredient a polypeptide of this invention in a pharmaceutically acceptable carrier, exipient or stabilizer, and optionally other therapeutic constituents. Acceptable carriers, exipients or stabilizers are non-toxic to recipients at the dosages and concentrations employed, and inculde buffers, such as phosphate buffered saline and like physiologically acceptable buffers, and more generally all suitable carriers, exipients and stabilizers known in the art.

## EXAMPLES

### Example 1 - Binding of OGP to OGPBP Materials

t-Boc-Gly, $OCH_2$-Pam resin, N-Boc protected amino acid derivatives, N,N-dicyclohexylcarbodiimide (DCC), 1-hydroxy-benzotriazole (HOBt), diisopropyl-ethylamine (DIEA), tri-fluoroacetic acid (TFA), N,N-dimethylformamide (DMF) and dichloromethane (DCM) were obtained from Applied Biosystems Inc. (Foster City, CA). Hydrogen fluoride (HF) was purchased from Matheson (secacuse, NJ), Boc-3-I-Tyr-(BZ1)-OH from Bachem (Torrance, CA), p-cresol from Aldrich Chemical Co. (Milwaukee, WI) and sephadex G15F from Pharmacia (Uppsala, Sweden). N-ethylmaleimide (NEM) was from sigma Chemical Co. (St. Louis, MO). Polyacrylamide, N,N,N',N'-tetramethylenediamine (TEMED) and ammonium persulfate (APS) were from BioRad Laboratories (Richmond, CA). Na[$^{125}$I] was from Nuclear Research Center (Negev, Israel). Iodogen was from Pierce (Pierce Chemical Company, Rockford, IL). Sabra mice were from the breeding facility of the Hebrew University School of Medicine.

### Methods

#### Preparation of sOGP and OGP analogues [$Cys^{15}$]OGP-$NH_2$, [Ac-$Cys^0$]OGP and [$Tyr^{10}$(3-I)]OGP

sOGP was synthesized by the solid phase method of Merrifield [Merrifiled, (1969)Adv. Enzymol. 32:221]) using an Applied Biosystems Model 430A Automated Peptide Synthesizer (Applied Biosystems Inc. Foster City CA). The synthesis was carried out on 0.5 mmol t-Boc-Gly-$OCH_2$-Pam resin (1% cross-linked, 0.78 mmol/g). The amino acid derivatives were protected on the $\alpha$-amino function by t-butyloxycarbonyl (Boc) groups. Protection of the side chains was as follows: Arg (Tos), Lys (2-Cl-Z), Tyr (2-Br-Z), cys (4-Mc BZl) and Thr (O-Bzl). Coupling of the Boc protected derivatives of Arg and Gln was by the DCC-HOBt method of Konig, W. and Geiger, R. [Chem. Ber. 103:788 (1970)]. All other amino acid derivatives were coupled via the DCC-mediated preformed symmetrical anhydride method of Hagemaier, H. and Frank, H. [Hoppe-Seyler's Z. Physiol. Chem. 353:1973 (1972)]. The coupling of each amino acid residue was repeated twice. Deprotection of the blocked amino-terminus was by treatment with 60% TFA in DCM. Side chains were deprotected and the peptide was cleaved from the resin (2.7 g resin-bound peptide) using the HF procedure where a mixture of 4 ml anisole and 36 ml liquid HF was used for 75 min at 0°C. The crude synthetic peptide was partially purified on a Sephadex G15F 3 x 35 cm column, eluted with 50% (v/v) aqueous acetic acid. Further purification was accomplished on a Waters DeltaPrep 3000 HPLC instrument equipped with a PrePak Vydac protein C18 column (15 $\mu$, 5.5 x 35 cm) (The Separation Group, Hesparia, CA). The cartridge was pumped with 5-33.5% acetonitrile linear gradient containing 0.1% TFA at a flow rate of 100 ml/min.

Preparation of [$Cys^{15}$(NEM)]OGP-$NH_2$

[$Cys^{15}$(NEM )]OGP-$NH_2$, is a synthetic OGP analogue extended at the C-terminal with Cys[S-2-(N-ethylsuccinyl)]-carboxamide. 100 mg of [$Cys^{15}$]OGP-$NH_2$ obtained after the HF procedure were dissolved in 2 ml of 0.5 M PBS, pH 7.0, containing 100 mg N-ethyl maleimide and 0.6 ml dimethylformamide and allowed to stir at room temperature for 1 h. The reaction mixture was diluted with 3 ml of 0.1% TFA in water and filtered through a 0.45 $\mu$m pore size millipore membrane. The crude [$Cys^{15}$(NEM)]OGP-$NH_2$ thus obtained was purified by loading this solution onto a preparative HPLC Vydac Protein C18 reverse phase column employing a linear gradient 2 1 0-40% acetonitrile at a flow rate of 100 ml/min (Fig. 1a). The homogeneity and identity of the purified preparation was confirmed by fast atom bombardment mass spectrometry (FAB-MS, MW=175l). In further purification cycles the identity and purification of the [$Cys^{15}$(NEM)]OGP-$NH_2$ peak were determined using analytical HPLC RP-18, C18 reverse-phase column (Merck, Darmstadt, FR Germany) pumped with 30 ml 20-33% acetonitrile gradient at a flow rate of 1 ml/min. The elution position and homogeneity of the purified peak (Figs. 1a and 1d) were compared to those of the standard preparation tested by FAB-MS (Fig. 1c). The fractions containing the purified [$Cys^{15}$(NEM)]OGP-$NH_2$ (Figs. 1a and 1d) were pooled, dried and the pure solid peptide was stored at -70°C.

Preparation of [Ac-$Cys^0$(NEM)]OGP-$NH_2$

This analogue was prepared by extending the sOGP N-terminus with acetyl-Cys[S-(N-ethylsuccinyl)]-carboxamide using a similar procedure described above for [$Cys^{15}$(NEM)]OGP-$NH_2$.

Radioiodination

Radioiodination of sOGP was carried out in 70 $\mu$l reaction mixture containing 25 $\mu$g sOGP, 5 $\mu$g Iodogen and 2 mCi Na[$^{125}$I] in 100 mM phosphate buffer, pH 7,5. The mixture was left for 5 min at room temperature and the iodination stopped by adding 200 $\mu$l water. The unreacted iodide was removed using a reverse phase SEP-PAK C18 cartridge (Waters Associates, Milford, MA) (Fig 2). The Na[$^{125}$I] was washed out with 100 mM phosphate buffer pH 7.5. The radioiodinated peptide was eluted with 50% acetonitrile/0.1% TFA in aqueous solution, resulting in a single sharp peak of radioactivity (Fig. 2). Fractions comprising this peak were pooled and concentrated by evaporation in a SpeedVac dryer (Savant Instruments Inc., Farmingdale, NY). Further purification was carried out on HPLC reverse phase Vydac C4 column eluted with 30 ml 16-23% acetonitrile gradient at a flow rate of 1 ml/min. The position of the purified peak was determined by a test run of 5 $\mu$l aliquot to which 0.25 $\mu$g was added. Fractions of the peak that co-eluted with the cold [$Tyr^{10}$(3-I)]OGP (Fig. 3) were pooled, diluted with an equal volume of 0.05 M HEPES buffer, pH 7.4, and stored at -70°C until use.

Binding assay

[$Tyr^{10}$(3-$^{125}$I)]sOGP, $10^5$ cpm/ml, was incubated for 30 min at 37°C with 1:50 dilution in PBS of fresh serum obtained from 300 g male Sabra rats or volunteer human donors. The incubation was carried out in the absence of unlabeled peptides or in the presence of 0.0625- 6.25 nmol/ml sOGP, rOGP, [Ac-$Cys^0$(NEM)]-OGP, or [$Cys^{15}$(NEM)]OGP-$NH_2$. The bound and unbound [$Tyr^{10}$(3-$^{125}$I)]OGP were separated using a cathodic native PAGE system, run in a Biorad Minigel apparatus. The separating gel consisted of 15% polyacrylamide, suspended in 0.1 M phosphate buffer pH 6.0 containing 13% glycerol, 0.1% TEMED and 0.1% APS. The stacking gel consisted of 4% polyacrylamide, 0.03 M phosphate buffer pH 6.8 containing 0.1% TEMED and 0.1% APS. Cathodic electrophoresis was carried out in 1 mM phosphate buffer pH 6.0, at 100 volt for 30 min followed by 3 h at 150 volt. The gels were dried and autoradiographed by exposure to Agfa Curix RP-2 film at -70°C.

Results

$[Tyr^{10}(3-^{125}I)]$OGP was competed out from the $[Tyr^{10}(3-^{125}I)]$OGP-OGPBP complex, dose dependently, by cold sOGP with maximal displacement occurring at sOGP concentrations higher than 300 nmol/ml undiluted serum (as shown for rat serum in Fig. 4). The displacement effect was mimicked by $[Cys^{15}(NEM)]OGP-NH_2$. $[Ac-Cys^{0}(NEM)]$-OGP failed to displace the radioiodinated peptide from the $[Tyr^{10}(3-^{125}I)]$OGP-OGPBP complex (Fig. 4), indicating a role for the OGP N-terminal region in the OGP-OGPBP complex formation. The proteinous nature of the OGPBP was demonstrated by inhibition of the complex formation following trypsin or thermolysin digestion prior to the incubation with $[Tyr^{10}(3-^{125}I)]$sOGP.

Example 2 - Measuring irOGP in Biological Fluids Materials

Bradykinin, substance P, parathyroid hormone (PTH) (1-34), BSA (Cat No. A-7030), Tween 20, p-nitrophenyl phosphate (Sigma 104) and diethanolamine buffer pH 9.6, were purchased from Sigma Chemical Co. (St. Louis, MO). Histone H4 (HH4) was from Boehringer Mannheim, Germany). Microtiter plates were from Dynatech (Denkendorf, Germany). Goat alkaline phosphatase conjugated anti-rabbit IgG (heavy and light chains) were obtained from BioMakor (Rehovot, Israel). Tissue ingredients were purchased from Biological Industries (Beith Haemek, Israel). 16 mm multiwell dishes were from Nunc (Nunc, Roskilde, Denmark).

Methods

Preparation of anti-OGP IgG

Anti-OGP antiserum was generated in rabbits in the standard manner using maleimido modified KLH which was conjugated with either $[Ac-Cys^{0}]$OGP or $[Cys^{15}]OGP-NH_2$, as before [Lerner et al. (1981) Proc. Natl. Acad. Sci. USA 78:3403]. The IgG fraction was collected from the antiserum by ammonium sulfate fractionation. In ELISA this antibody preparation reacted specifically with the OGP C-terminal region as revealed from its binding to $[Ac-Cys^{0}(NEM)]OGP-NH_2$ and failure to bind to $[Cys^{15}(NEM)]OGP-NH_2$ and $[Tyr^{10}(3-I)]$OGP. In addition, the anti-OGP IgG failed to react with HH4, PTH (1-34), bradykinin and substance P.

Competitive ELISA

Dynatech microtiter plates were coated with 100 μl of 10 μg/ml sOGP in 0.5 M sodium carbonate buffer pH 9.5, overnight at 4°C. The wells were washed with 200 μl PBS (x3). Blocking of unreacted sites on the plastic well bottom was carried out by incubation of 1% gelatine in PBS at 37°C for 1 h. Rinsing with PBS (3 x 200 μl) was followed by 2 h incubation with the anti-OGP IgG preparation (diluted 1:300 with reference to the original rabbit anti-serum) at room temperature. This was followed by rinsing with (i) 3% BSA in PBS (1 x 200 μl); (ii) 0.02% Tween 20 in PBS (1 x 200 μl); and (iii) PBS (3 x 200 μl). The wells were then incubated with 100 μl of 1:1000 dilution in PBS of goat alkaline phosphatase conjugated anti-rabbit IgG for 2 h at room temperature. Rinsing with (i) 0.02% Tween in PBS (1 x 200 μl); (ii) PBS (2 x 200 μl); (iii) bidistilled water (3 x 200 μl) preceded a 20 min incubation at room temperature with alkaline phosphatase buffer solution: p-nitrophenyl phosphate 1 mg/ml in substrate buffer (diethanolamine buffer pH 9.6). Light absorbance at 450 nm was then recorded using an ELISA reader. In competitive ELISA the anti-OGP IgG was preincubated for 30 min at 37°C with the respective test sample or samples of known sOGP content for calibration. All samples were tested in duplicates from which the background reading of controls, done in wells uncoated with sOGP, was subtracted. Steady state irOGP in biological fluids was measured by preincubating diluted, but otherwise untreated, samples. For measurements of total irOGP this preincubation was preceded by reaction with 450 nmol/ml-undiluted serum $[Cys^{15}(NEM)]OGP-NH_2$ for 30 min at 37°C.

Binding capacity of the OGPBP

The binding capacity of the OGPBP in sera and tissue culture media was determined by adding increasing concentrations of sOGP to-, and measurement of irOGP in-, aliquots of the respective test samples. The binding capacity was calculated according to the equation:

Binding Capacity = Max[Total irOGP + added sOGP - measured irOGP]

as shown in Fig. 5.

Cell cultures

Cells in the test cultures were seeded in 16 mm multiwell dishes at 2 x $10^4$ cells/well and incubated at 37°C in $CO_2$-air. ROS 17/2.8 were maintained in F12 medium supplemented with 10% fetal calf serum (FCS) and subcultured once a week. MC3T3 E1 and NIH 3T3 cells were maintained in αMEM supplemented with 10% FCS and subcultured twice a week. Cells for experiments were derived from maintenance cultures at confluency. For each cell type the cultures were prepared in the same respective medium. For the initial 46 h the medium was supported with 2% and 10% FCS in the ROS and 3T3 cells, respectively. Incubation with 4% BSA was preceded by washing of the cells with PBS, 2 h incubation with serum-free medium, and washing with medium. Steady state irOGP, total irOGP and cell number were determined prior to the last washing and 12, 24, 36 and 48 h after the addition of BSA.

Marrow ablation

Ablation of tibial marrow was carried out in one limb of 230-250 g male rats of the Hebrew University-Sabra strain as before [Bab et al. (1985) Calcif. Tissue Int. 37;551]. Blood was drained from the tail immediately prior to ablation and 1, 5, 10 and 12 days thereafter. The resultant serum samples were collected and frozen at -70°C. The samples were thawed simultaneously and assayed as described above for total and steady state irOGP.

Total body irradiation

C57 black female mice weighing 15-20 g were subjected to a single, 900 rad, total body x-ray irradiation. Blood was drawn from the heart 5, 10 and 12 days after irradiation, from 5 animals at each time period. In addition, blood was similarly obtained from an untreated group of 5 animals designated time group "0". Total and steady state irOGP levels in the blood samples were determined as above.

Effect of exogenously administered sOGP on engraftment of bone marrow transplants in irradiated mice

C57 black female mice weighing 15-20 g were divided into the following treatment groups, each consisting of 5 animals:

**Group 1** received sOGP, as a solution in PBS, 5 ng/mouse/day, intravenously for 9 days. On day 4 the animals were subjected to a single 900 rad total body x-ray irradiation. on day 5 the animals were given a syngeneic marrow transplant, consisting of $10^6$ unselected cells. The animals were killed 10 days after transplantation and blood was drawn as above for total and steady state irOGP determination. In addition, total marrow and spleen cellularity were assessed by direct cell counts in respective cell suspensions.

**Group 2** received the same treatment. However, the administration of exogenous sOGP was stopped after the first three daily injections.

**Group 3** animals served as controls, sham injected with peptide-free PBS for 9 days.

Results

The standard curve obtained by applying the competitive ELISA assay to solutions containing known amounts of sOGP in PBS demonstrates that concentrations as low as 0.25 ng/ml and as high as 10 ng/ml irOGP can be efficiently measured with this assay (Fig. 6).

To determine the concentration of [$Cys^{15}$(NEM)]OGP-$NH_2$ required for complete displacement of irOGP from its complex with OGPBP, increasing concentrations of this analogue were reacted with biological fluids that show endogenous steady state irOGP: human, mouse and rat serum and medium from cultures of ROS 17/2.8, MC 3T3 E1 and NIH 3T3 cells. In all these biological fluids [$Cys^{15}$(NEM)]OGP-$NH_2$ concentration of 450 nmol/ml or higher revealed maximal irOGP readings (Fig. 7). This indicates that [$Cys^{15}$(NEM]OGP-$NH_2$ at 450 nmol/ml concentration is useful for the determination of total irOGP.

The ranges of both steady state and total irOGP, as well as the binding capacity of the OGPBP, were similar in human, rat and mouse serum as shown in Table A.

TABLE A

| STEADY STATE, TOTAL IMMUNOREACTIVE OGP AND BINDING CAPACITY OF OGPBP IN NORMAL SERA | | | |
|---|---|---|---|
| **Species** | **Steady State** nmol/ml | **Total** nmol/ml | **Binding Capacity** nmol/ml |
| Human | 0.41-1.08 | 10.33-23.0 | 176 |
| Rat | 0.27-0.44 | 7.40-13.54 | 162 |
| Mouse | 0.26-0.31 | 7.70-13.0 | 158 |

Table B shows that highest levels of both steady state and total irOGP were found in the medium from cultures of transformed osteogenic ROS 17/2.8 cells. Lowest levels were found in medium from fibroblastic NIH 3T3 cultures with the medium from the osteogenic MC 3T3 E1 cell cultures showing intermediate levels. The binding capacity of the OGPBP in the medium from NIH 3T3 cell cultures was approximately twice as that of media from ROS 17/2.8 or MC 3T3 E1 cells. The steady state levels in all three cultures showed a progressive increase throughout the 48 h experiment period. The level of the total irOGP increased during the first 24 h and decreased thereafter when the cultures reached confluency. This inverse relationship between the steady state and total levels of irOGP indicates a decrease in the binding capacity of the OGPBP.

TABLE B

STEADY STATE AND TOTAL IMMUNOREACTIVE OGP IN TISSUE CULTURE MEDIA

| Medium | Steady State nmol/$10^6$ cells | Total nmol/$10^6$ cells | cells/$cm^2$ |
|---|---|---|---|
| ROS 17/2.8* | | | |
| 12 h | 0.027 | 102 | 26,320 |
| 24 h | 0.052 | 255 (820)** | 23,800 |
| 36 h | 0.065 | 178 | 22,520 |
| 48 h | 0.105 | 120 | 29,000 |
| MC 3T3 E1 | | | |
| 12 h | 0.0018 | 59 | 35,400 |
| 24 h | 0.0041 | 97 (623)** | 30,800 |
| 36 h | 0.0064 | 65 | 38,200 |
| 48 h | 0.0098 | 35 | 53,000 |
| NIH 3T3 | | | |
| 12 h | 0.002 | 22 | 112,400 |
| 24 h | 0.0034 | 36 (349)** | 113,600 |
| 36 h | 0.0039 | 28 | 123,200 |
| 48 h | 0.0065 | 20 | 148,000 |

* Time periods after cell washing and addition of BSA.
**The numbers in brackets indicate the binding capacity.

Fig. 8 shows a marked increase in steady state and total irOGP levels in rat serum during the first ten days of post-ablation marrow regeneration in the tibia. From day 10 to day 12 there was a dramatic drop of the total irOGP to normal with the steady state levels of approximately half the pre-ablation level, indicting an increase in the binding capacity of the serum OGPBP. Fig. 9 shows that 12 days after total body irradiation in mice there was a parallel increase in steady state and total serum irOGP. In irradiated mice that received a marrow transplant, the steady state and total serum OGP responded to daily administered exogenous sOGP by an increase that correlated positively with the length of period of administration (Fig. 10) In the same animals there was a strong and highly significant correlation between the total irOGP and the cellular content of the spleen (Fig. 11). The marrow cellularity did not reveal such a correlation.

## Claims

1. A polypeptide which binds to naturally occurring osteogenic growth-polypeptide binding protein/s and does not bind to an antibody directed against said naturally occurring osteogenic growth polypeptide, said osteogenic growth polypeptide-binding proteins being proteins which bind to the N-terminal region of osteogenic growth polypeptide and to analogues thereof.

2. A polypeptide according to claim 1 which binds through its N-terminal region to said osteogenic growth polypeptide-binding protein/s.

3. A polypeptide according to claim 1 or claim 2 being a modified osteogenic growth polypeptide.

4. A polypeptide according to claim 3 in which the C-terminal region is modified to prevent binding of the said polypeptide to an antibody directed against osteogenic growth polypeptide.

5. A polypeptide according to claim 3 or claim 4 having the formula [$Cys^{15}$(NEM)]OGP-$NH_2$ as herein defined.

6. A polypeptide according to claim 3 or claim 4 having the formula [$Tyr^{10}$(3-I)]OGP as herein defined.

7. A polypeptide according to claim 1 which binds to a naturally occurring osteogenic growth polypeptide-binding protein and does not bind to an antibody directed against osteogenic growth polypeptide which antibody binds to only the C-terminal region of osteogenic growth polypeptide.

8. A polypeptide according to claim 7 wherein said antibody binds to [Ac-$Cys^{0}$(NEM)]OGP-$NH_2$ as herein defined but does not bind to [$Cys^{15}$(NEM)]OGP-$NH_2$ and [$Tyr^{10}$(3-I)]OGP.

9. A method of releasing an immunoreactive osteogenic growth polypeptide from its complex with osteogenic growth polypeptide-binding protein/s wherein the said complex is reacted with a polypeptide according to claim 1, whereby the immunoreactive osteogenic growth polypeptide is competed out of said complex by the polypeptide according to claim 1.

10. A method according to claim 9 wherein said polypeptide is [$Cys^{15}$(NEM)]OGP-$NH_2$.

11. A method according to claim 9 wherein said polypeptide is [$Tyr^{10}$(3-I)]OGP.

12. A method of quantitatively determining the total immuno-reactive osteogenic growth polypeptide content in a sample of a biological fluid or tissue extract comprising the steps of:
(a) reacting said sample with a polypeptide according to claim 1 under suitable reaction conditions;
(b) incubating the reaction mixture obtained in step (a) with an antibody directed against osteogenic growth polypeptide under suitable incubation conditions; and
(c) calculating the amount of immunoreactive osteogenic growth polypeptide which was present in said sample from the amount of free or bound antibody as measured by immunoassay techniques.

13. A method according to claim 12 wherein the polypeptide employed in step (a) is a modified osteogenic growth polypeptide.

14. A method according to claim 13 wherein said osteogenic growth polypeptide is modified at the C-terminal region thereof to prevent its binding to an antibody directed against naturally occurring or synthetic osteogenic growth polypeptide.

15. A method according to any one of claims 12 to 14 wherein the polypeptide employed in step (a) is [$Cys^{15}$(NEM)]OGP-$NH_2$.

16. A method according to any one of claims 12 to 14 wherein the polypeptide employed in step (a) is [$Try^{10}$(3-I)]OGP.

17. A method for the determination of the binding capacity of osteogenic growth polypeptide-binding protein/s comprising the steps of:

(a) quantitatively determining the total amount of immunoreactive osteogenic growth polypeptide in an aliquot of a sample of a biological fluid or a tissue extract by the method of claim 12;
(b) incubating other aliquots of said sample each with a gradually increasing concentration of synthetic osteogenic growth polypeptide and quantitatively determining the amount of immunoreactive osteogenic growth polypeptide therein by immunoassay techniques; and
(c) calculating the binding capacity of the osteogenic growth polypeptide-binding protein/s in said sample by subtracting, for each of the aliquots of step (b), the amount of immunoreactive osteogenic growth polypeptide from the sums of the total immunoreactive osteogenic growth polypeptide determined in step (a) and the amount of synthetic osteogenic growth polypeptide added in step (b), wherein the maximal value obtained among said aliquots by said calculation represents the binding capacity of the osteogenic growth polypeptide-binding protein which was present in said sample.

**18.** A method of diagnosing pathological conditions associated with abnormal binding capacity of osteogenic growth polypeptide-binding protein/s by measuring the said binding capacity by the method of claim 17.

**19.** A method of diagnosing pathological conditions associated with abnormal bone formation by differentially determining in a sample of biological fluid or tissue extract from a patient suffering said condition, the binding capacity of osteogenic growth polypeptide by the method of claim 17, the total immunoreactive osteogenic growth polypeptide by the method of claim 12 and steady state immunoreactive osteogenic growth polypeptide by immunoassay techniques.

**20.** A method according to claim 19 for the diagnosis of metabolic bone diseases.

**21.** A method according to claim 20 for the diagnosis of osteoporosis, hyperparathyroidism, osteomalacia, rickets or renal osteodystrophy.

**22.** A method according to claim 19 for the early detection of osteogenic metastases associated with malignant diseases, particulaly prostate cancer.

**23.** A method according to claim 19 for evaluating the viability of bone marrow and prognosis of bone marrow transplants.

**24.** Pharmaceutical compositions containing as active ingredient a polypeptide according to claim 1, or a mixture of at least two such polypeptides, in a pharmaceutically acceptable carrier.

**25.** Pharmaceutical compositions according to claim 24 optionally containing further pharmaceutically acceptable agents.

**26.** Pharmaceutical compositions according to claim 24 or claim 25 wherein said polypeptide is $[Cys^{15}(NEM)]OGP-NH_2$.

**27.** Pharmaceutical compositions according to claim 24 or claim 25 wherein said polypeptide is $[Tyr^{10}(3-I)]OGP$.

Fig.1.

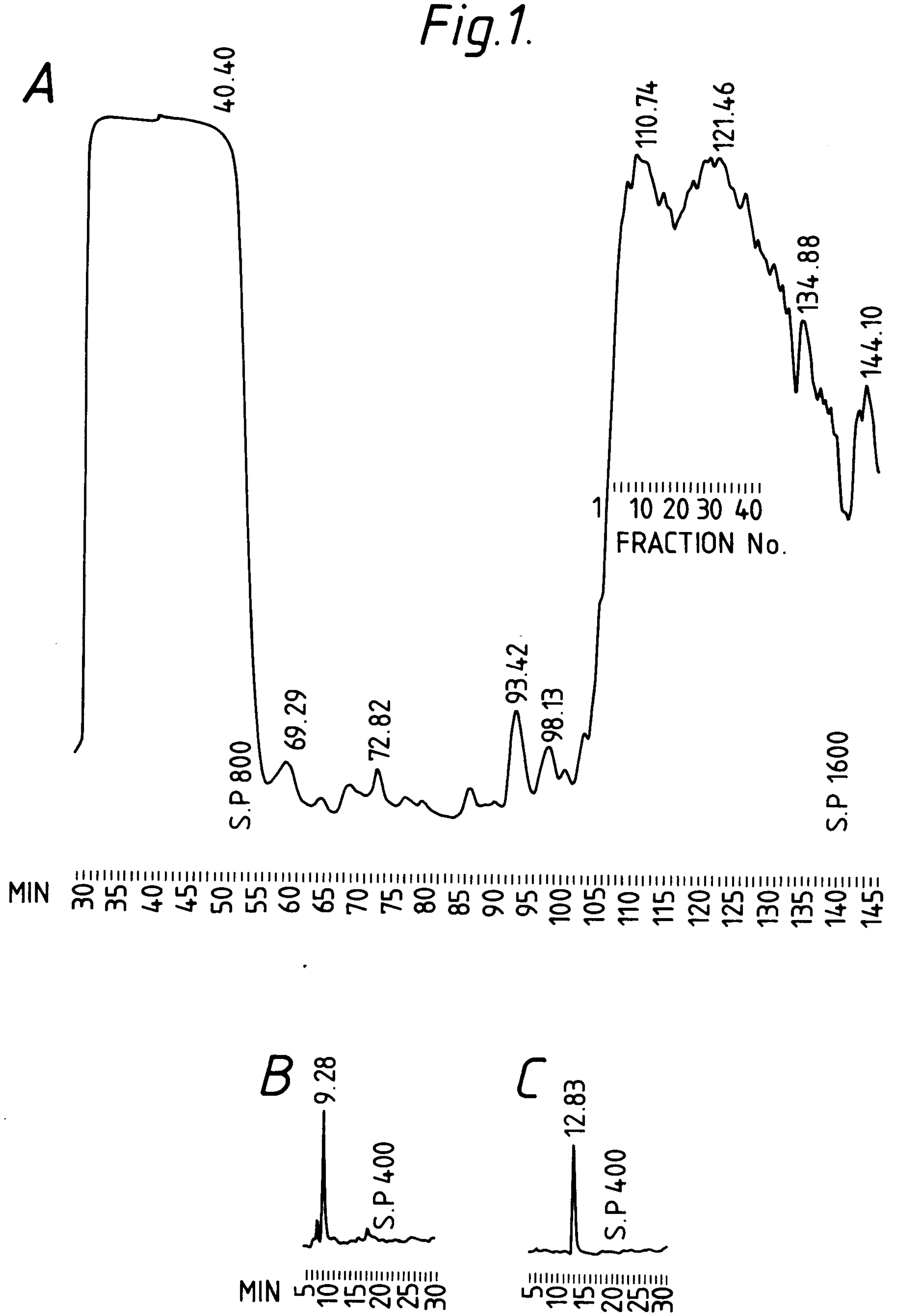
A
40.40
110.74
121.46
134.88
144.10
69.29
72.82
93.42
98.13
S.P 800
S.P 1600
1 10 20 30 40
FRACTION No.
MIN 30 35 40 45 50 55 60 65 70 75 80 85 90 95 100 105 110 115 120 125 130 135 140 145
B
9.28
S.P 400
MIN 5 10 15 20 25 30
C
12.83
S.P 400
5 10 15 20 25 30

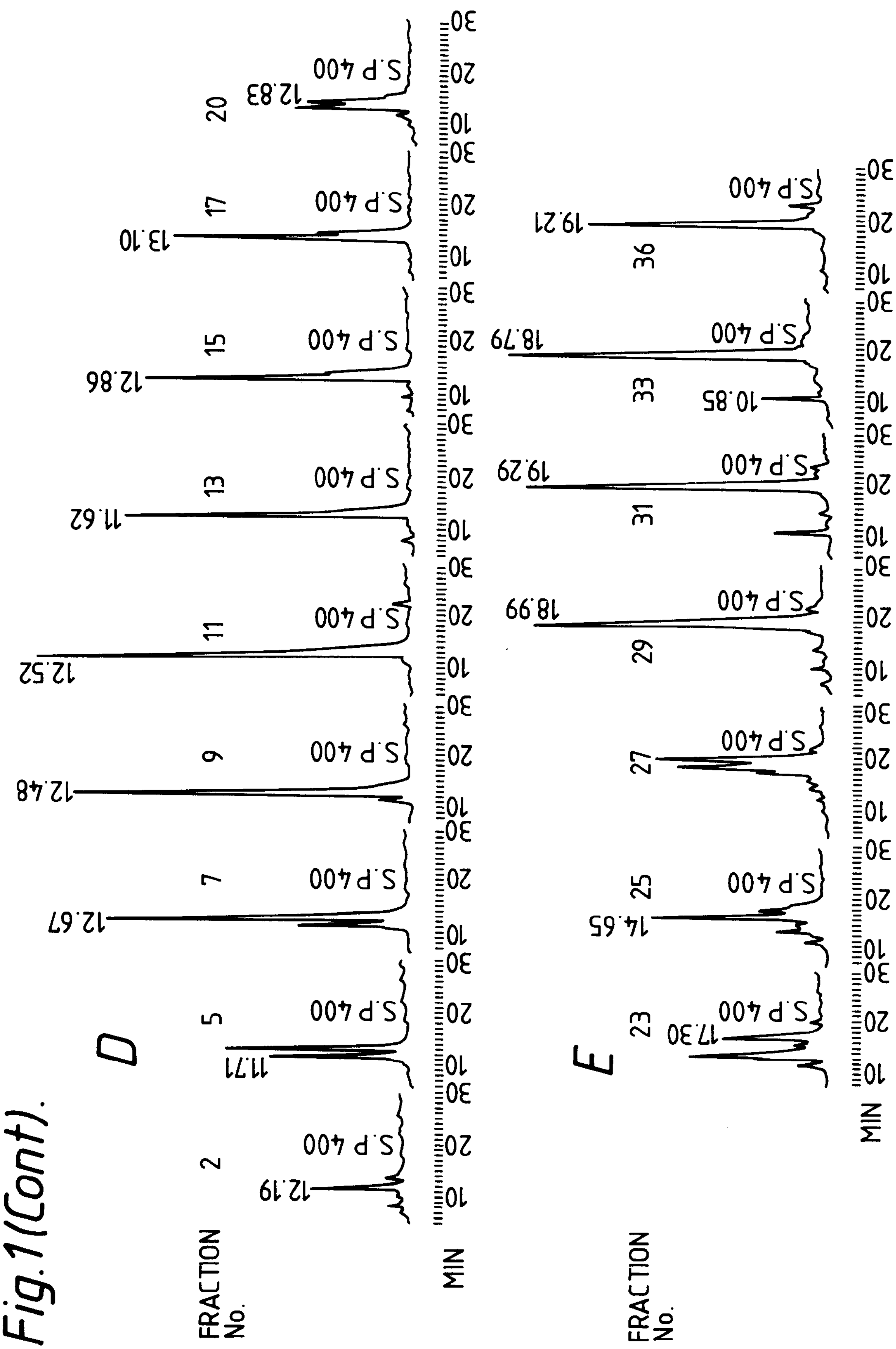
D
FRACTION No.
2
5
7
9
11
13
15
17
20
12.19
11.71
12.67
12.48
12.52
11.62
12.86
13.10
12.83
S.P 400
MIN
E
FRACTION No.
23
25
27
29
31
33
36
17.30
14.65
18.99
19.29
10.85
18.79
19.21
S.P 400
MIN

Fig.1(Cont).

Fig. 2.

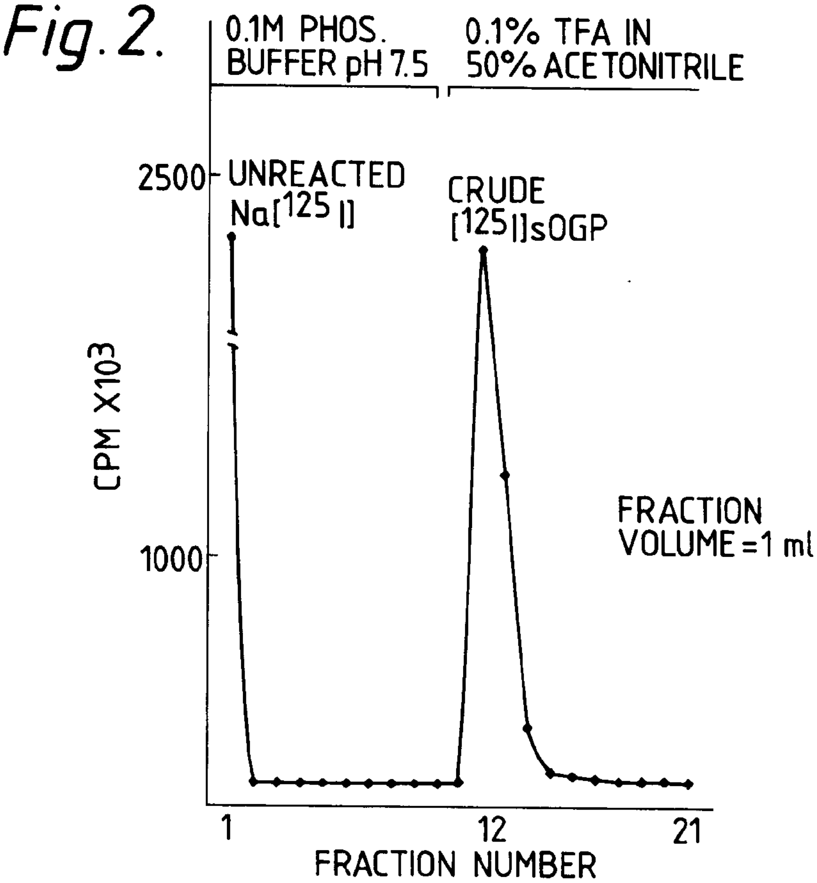
0.1M PHOS.
BUFFER pH 7.5
0.1% TFA IN
50% ACETONITRILE
2500
UNREACTED
Na[125 I]
CRUDE
[125 I]sOGP
CPM X103
FRACTION
VOLUME = 1 ml
1000
1
12
21
FRACTION NUMBER

Fig. 6.

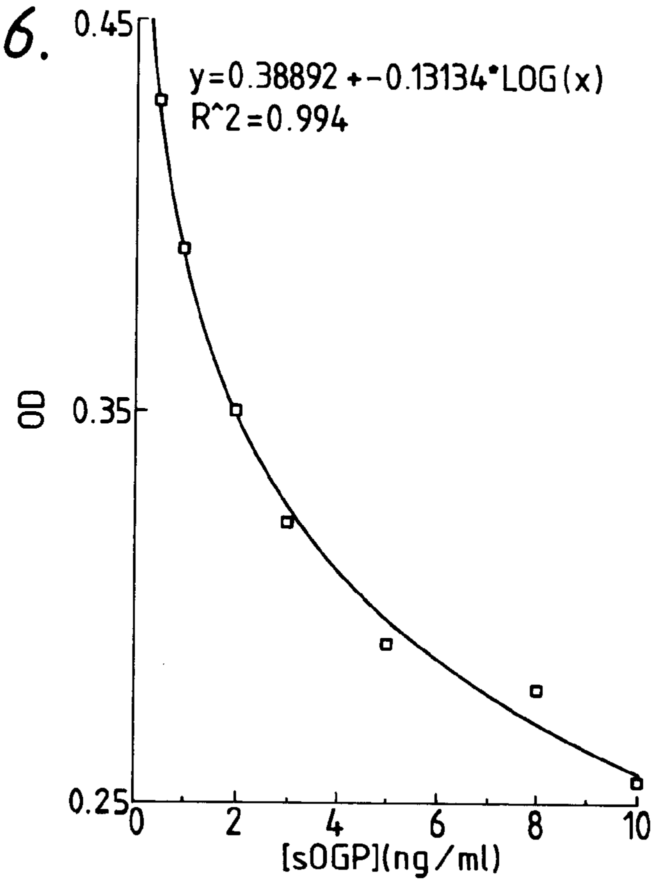
0.45
y=0.38892 + -0.13134*LOG(x)
R^2=0.994
OD
0.35
0.25
0
2
4
6
8
10
[sOGP](ng/ml)

*Fig. 3.*

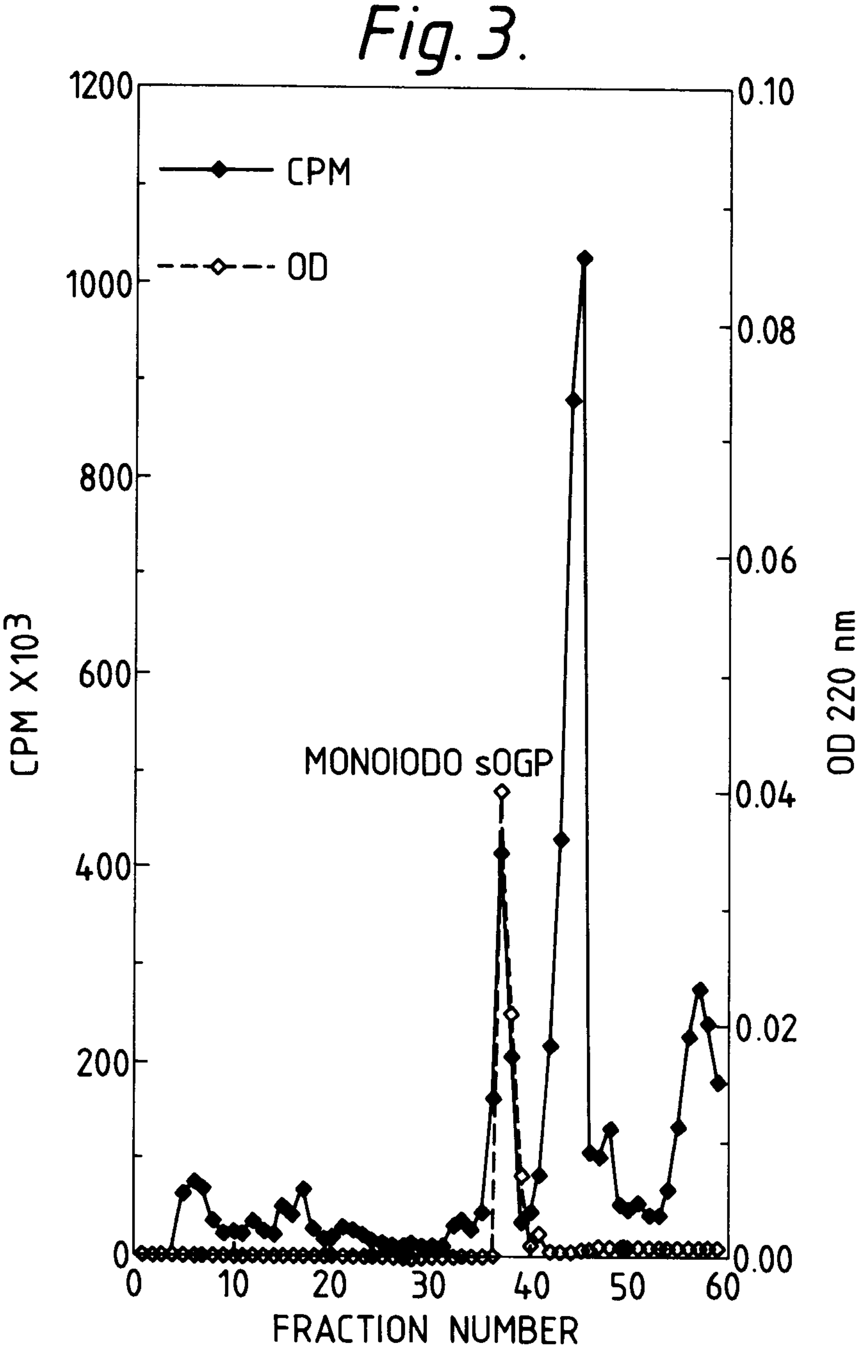


VYDAC PROTEIN C4 COLUMN
GRADIENT 16-23% ACETONITRILE
IN 0.1% TFA; 30 MIN
FRACTION VOLUME=0.5 ml
1ml / MIN

Fig.4.

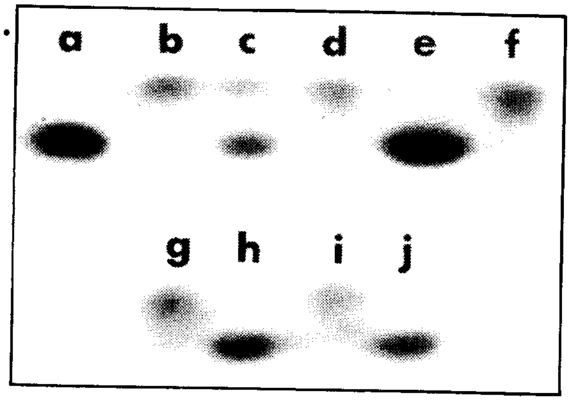
a
b
c
d
e
f
g
h
i
j

Fig.5.

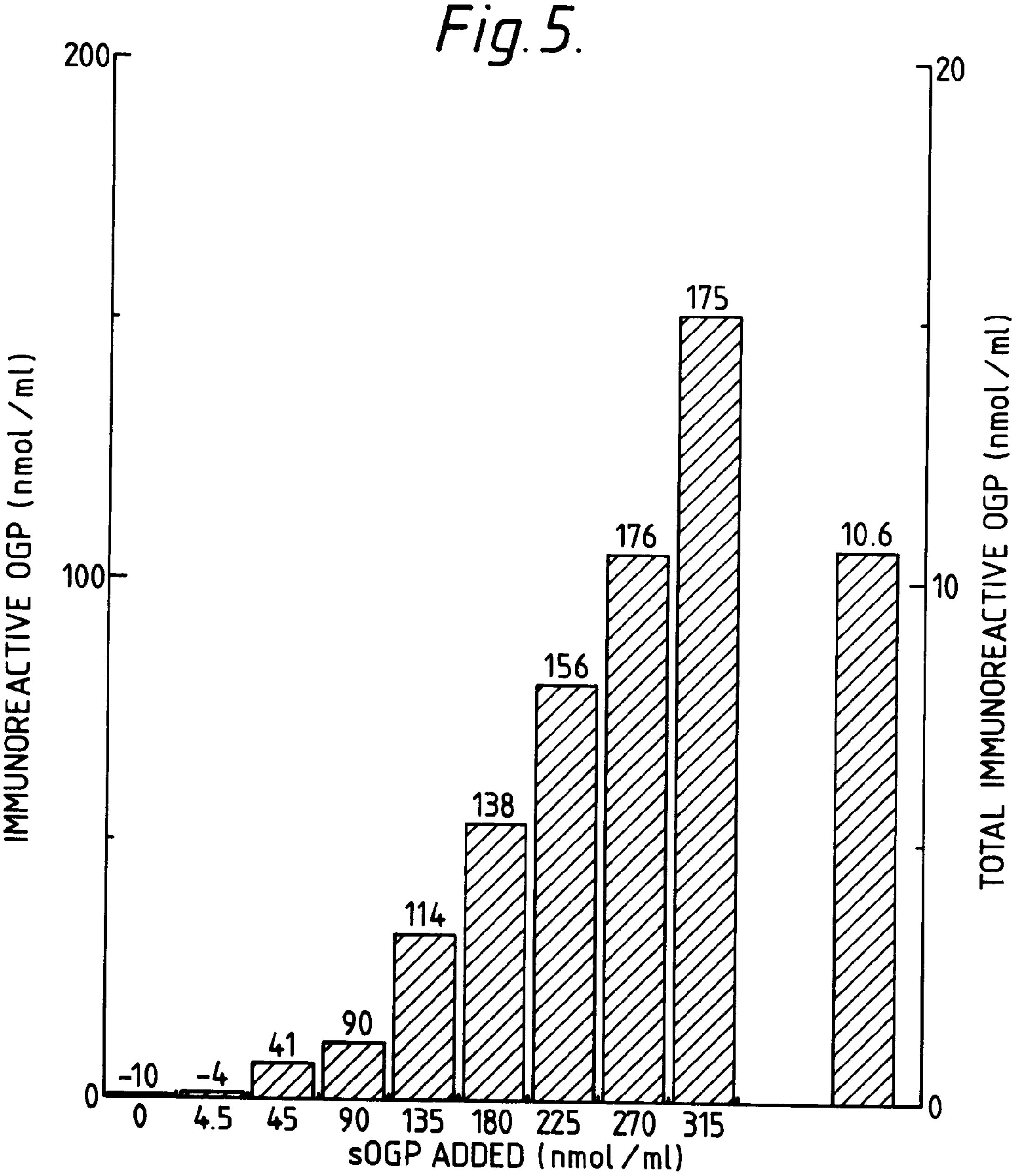
200
100
0
20
10
0
IMMUNOREACTIVE OGP (nmol / ml)
TOTAL IMMUNOREACTIVE OGP (nmol / ml)
-10
-4
41
90
114
138
156
176
175
10.6
0
4.5
45
90
135
180
225
270
315
sOGP ADDED (nmol / ml)

Fig. 7.

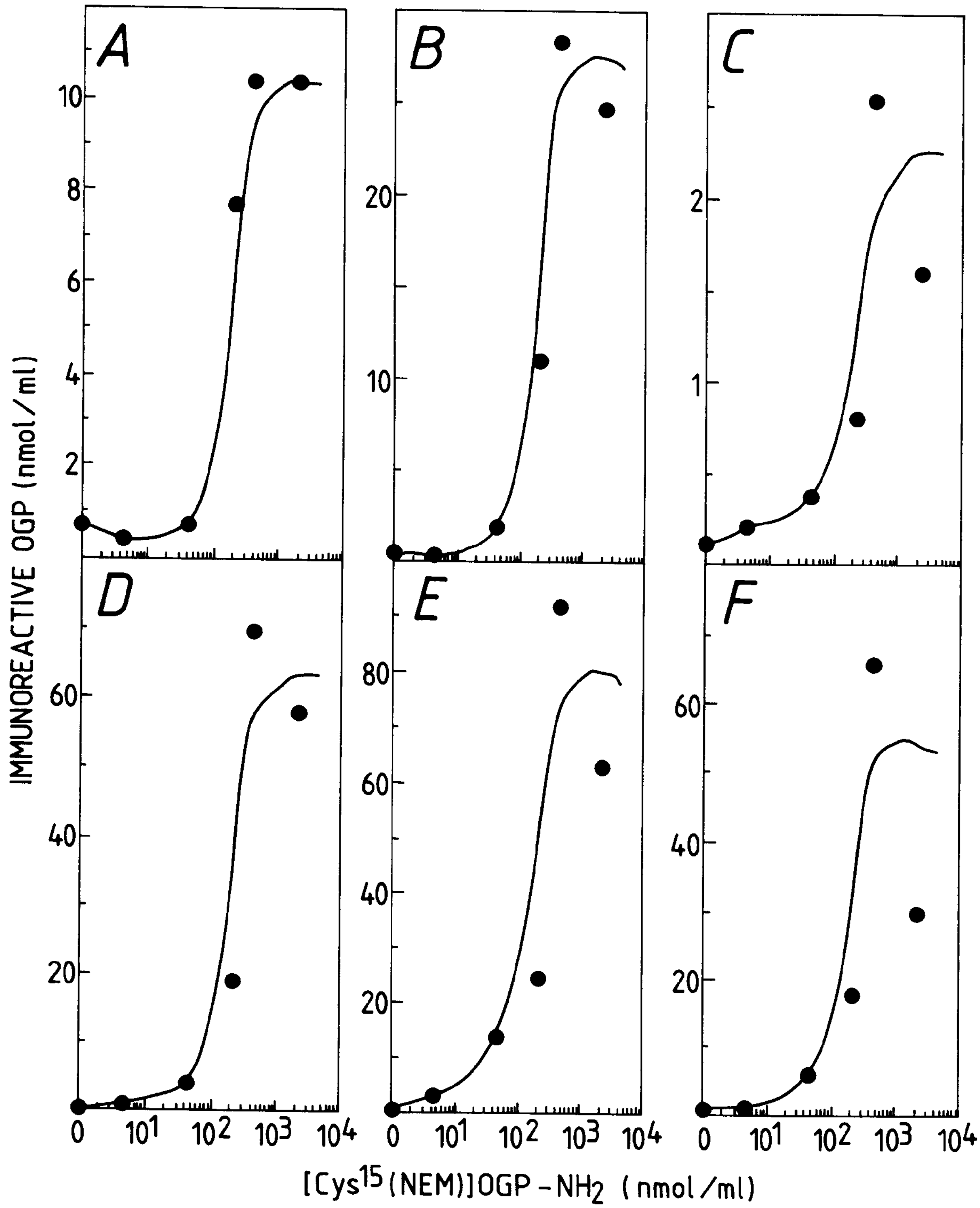
A
B
C
D
E
F
IMMUNOREACTIVE OGP (nmol/ml)
10
8
6
4
2
20
10
2
1
60
40
20
80
60
40
20
60
40
20
0
10^1
10^2
10^3
10^4
[Cys15(NEM)]OGP-NH2 (nmol/ml)

Fig.8.

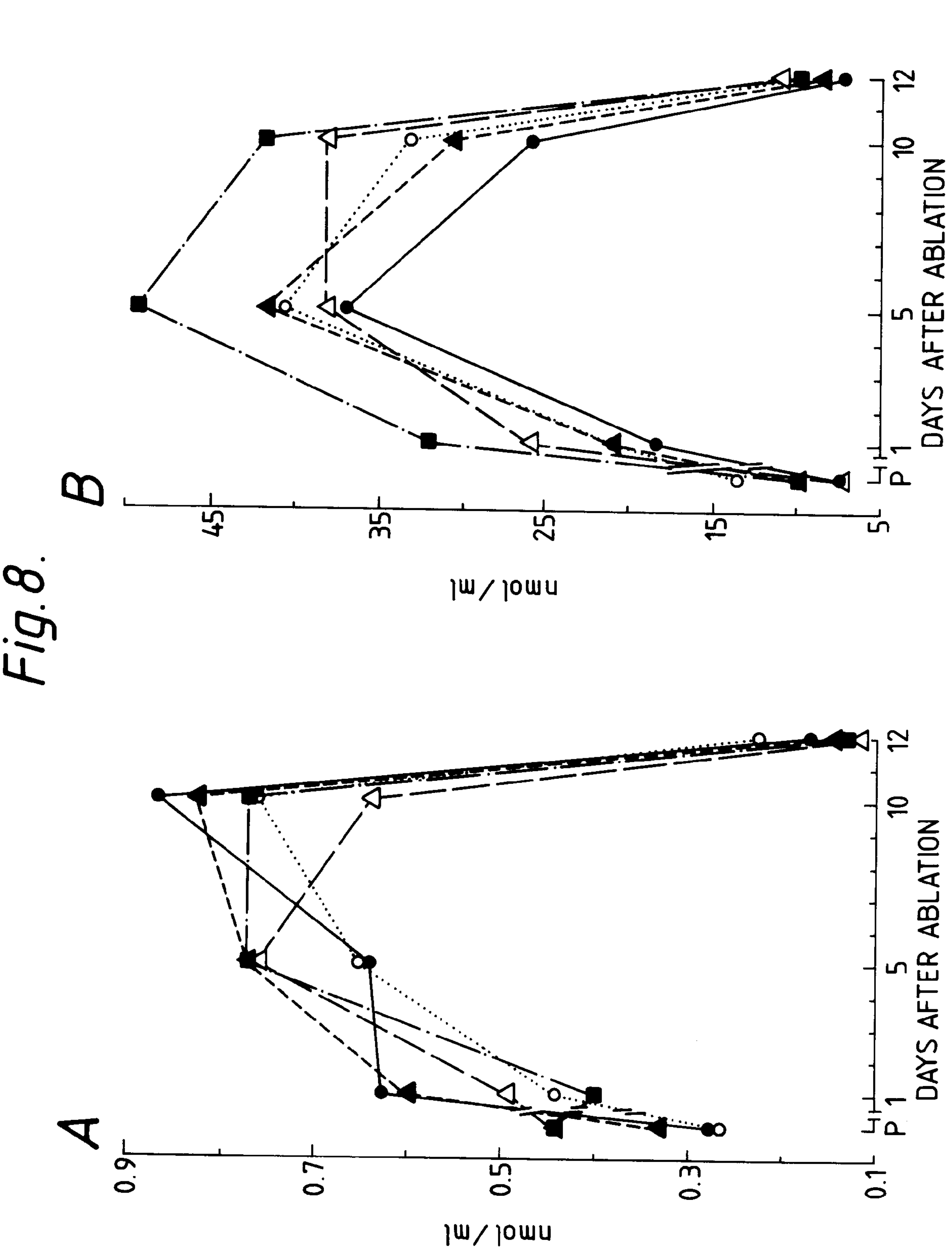
A
nmol / ml
0.9
0.7
0.5
0.3
0.1
P
1
5
10
12
DAYS AFTER ABLATION
B
nmol / ml
45
35
25
15
5
P
1
5
10
12
DAYS AFTER ABLATION

# Fig. 9.

EFFECT OF TOTAL BODY IRRADIATION ON IMMUNOREACTIVE OGP IN MOUSE SERUM

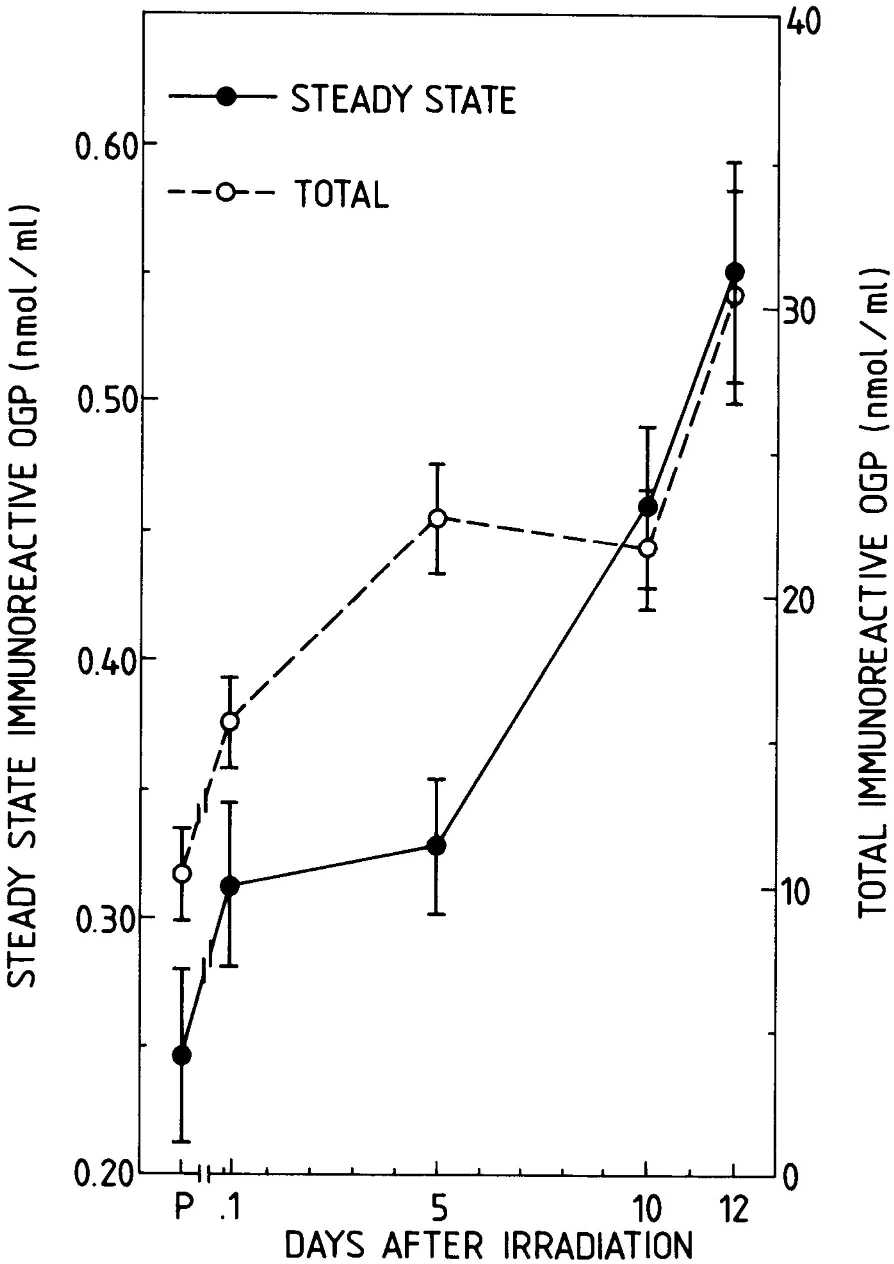

Fig.10.

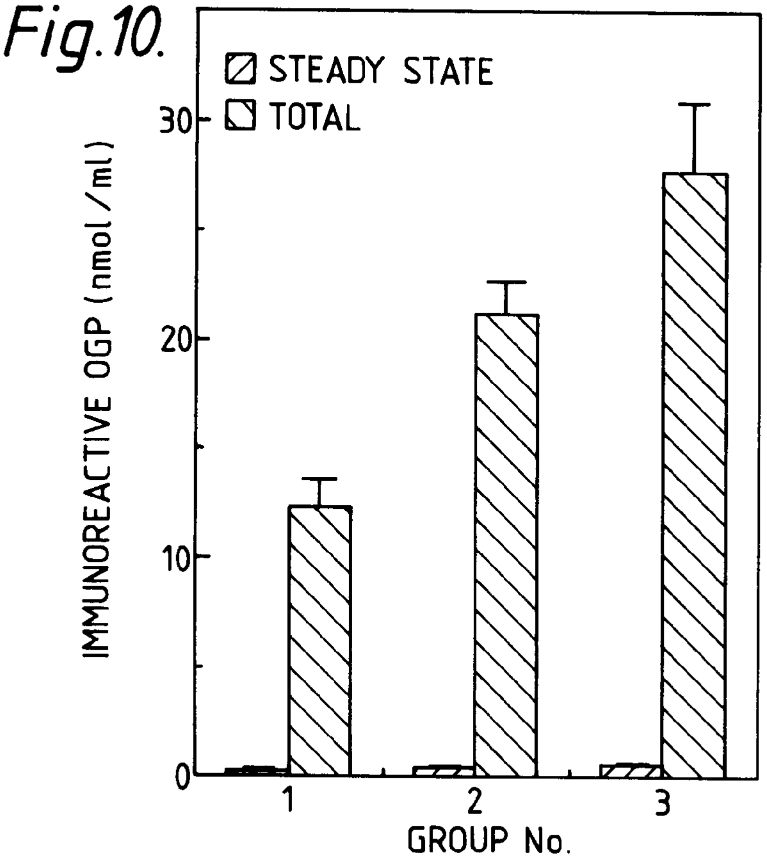
STEADY STATE
TOTAL
30
20
10
0
IMMUNOREACTIVE OGP (nmol / ml)
1
2
3
GROUP No.

Fig.11.

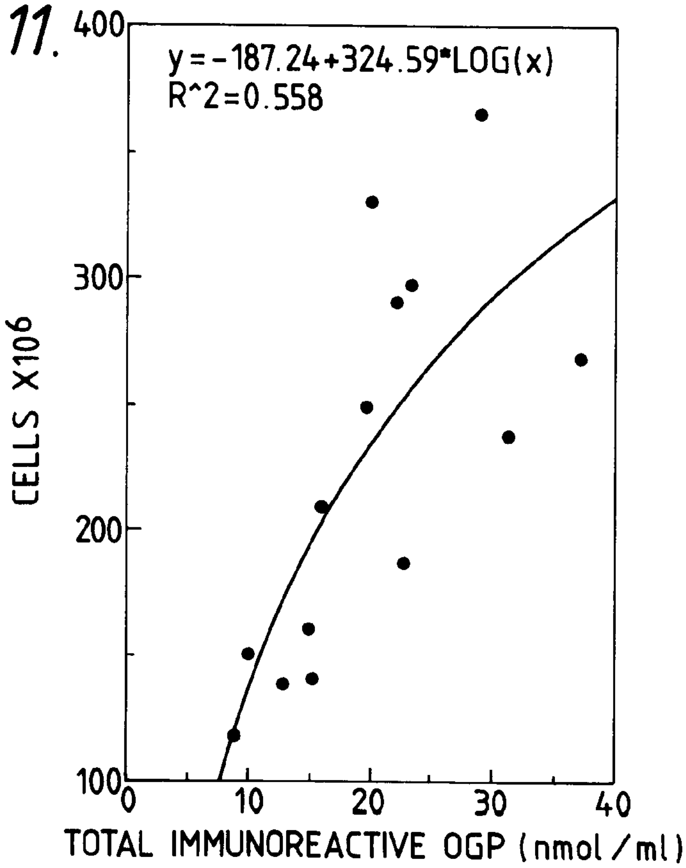
y=-187.24+324.59*LOG(x)
R^2=0.558
400
300
200
100
CELLS X106
0
10
20
30
40
TOTAL IMMUNOREACTIVE OGP (nmol / ml)

SEQUENCE LISTING

(1) GENERAL INFORMATION:

(i) APPLICANT:
(A) NAME: Yissum Research Development
Company of the Hebrew University of Jerusalem
(B) STREET: 46 Jabotinsky Street
(C) CITY: Jerusalem
(E) COUNTRY: Israel
(F) POSTAL CODE (ZIP): 91042

(ii) TITLE OF INVENTION: C-Terminal Modified Osteogenic Growth Polypeptides

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93303420.9

(2) INFORMATION FOR SEQ ID NO: 1:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO: 1:

```
Ala Leu Lys Arg Gln Gly Arg Thr Leu Tyr Gly Phe Gly Gly
1               5                   10
```

(2) INFORMATION FOR SEQ ID NO: 2:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Modified-site
(B) LOCATION: 15
(D) OTHER INFORMATION: /note= "Site 15 (Cys) is modified with the addition of (NEM)-NH2 wherein NEM signifies N-ethyl maleimide"

(xi) SEQUENCE DESCRIPTION: SEQ ID NO: 2:

```
Ala Leu Lys Arg Gln Gly Arg Thr Leu Tyr Gly Phe Gly Gly Cys
1               5                   10                  15
```

(2) INFORMATION FOR SEQ ID NO: 3:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Modified-site
(B) LOCATION: 10
(D) OTHER INFORMATION: /note= "Site 10 (Tyr) is modified with the addtion of (3-I)"

(xi) SEQUENCE DESCRIPTION: SEQ ID NO: 3:

```
Ala Leu Lys Arg Gln Gly Arg Thr Leu Tyr Gly Phe Gly Gly
1               5                   10
```